# EUROPEAN PATENT APPLICATION

(11) **EP 3 782 681 A1**
(43) Date of publication of application: **24.02.2021**
(21) Application number: 19787896.0
(22) Date of filing: 19.04.2019
(51) Int. Cl.: A61M 11/02

(54) **ATOMIZATION APPARATUS AND QUICK ATOMIZATION METHOD**

(30) Priority: 20.04.2018 CN 201810361578
(71) Applicant: Sunshine Lake Pharma Co., Ltd., Dongguan, Guangdong 523000 (CN)
(72) Inventor: CAO, Li, Dongguan, Guangdong 523871 (CN); ZHANG, Xuemei, Dongguan, Guangdong 523871 (CN); HU, Gang, Dongguan, Guangdong 523871 (CN); HUANG, Fangfang, Dongguan, Guangdong 523871 (CN)
(74) Representative: Hoffmann Eitle
(86) International application number: PCT/CN2019/083346
(87) International publication number: WO 2019/201324

(57) **Abstract**

An atomization apparatus and a quick atomization method. The atomization apparatus comprises: an atomization sheet for atomizing a drug and having a rated voltage; and a voltage control element, electrically connected to the atomization sheet, for providing a operating voltage for the atomization sheet, wherein the operating voltage is not less than 70 V, and the operating voltage is 60% to 150% of the rated voltage of the atomization sheet, or the ratio of the operating voltage to the rated voltage of the atomization sheet is greater than 100% and not greater than 150%. The quick atomization method comprises: providing an operating voltage to an atomization sheet, wherein the operating voltage is not less than 70 V, and the operating voltage is 60% to 150% of a rated voltage of the atomization sheet, or the ratio of the operating voltage to the rated voltage of the atomization sheet is greater than 100% and not greater than 150%. The atomization apparatus and atomization method can effectively improve the atomization speed of a drug.

## Description

### Field of the invention

The invention relates to the technical field of medical apparatus and instruments, in particular to an atomization apparatus and a quick atomization method.

### Background of the invention

With the continuous development and deepening of industrialization and urbanization, the air quality in cities is getting worse and worse, and people's respiratory diseases are becoming more common and frequent. On the contrary, people have higher and higher requirements for the quality of life. As a result, people want to have fast and effective methods and equipment for the treatment of respiratory diseases.

The medical instruments include an atomizer, which is mainly used to treat various upper and lower respiratory diseases, such as colds, fever, cough, asthma, sore throat, pharyngitis, rhinitis, bronchitis, and other diseases occurring in the trachea, bronchi, alveoli, and thorax such as pneumoconiosis. Atomization inhalation therapy is an important and effective treatment method for the treatment of respiratory diseases. The atomizer is used to atomize the liquid drugs into tiny particles. The drugs are deposited into the respiratory tract and lungs through breathing and inhalation to achieve painless, rapid and effective treatment.

The atomization sheet is one of the important components of the atomizer. The atomization sheet commonly used is mesh-type atomization sheet(also called microporous atomization sheet). The mesh-type atomization sheet is mainly composed of a piezoelectric ceramic sheet and a vibration sheet attached to the ceramic sheet. A lead is drawn from the piezoelectric ceramic sheet and the vibration sheet. During use, a voltage is applied to the atomization sheet. When the excitation electric signal is transmitted to the piezoelectric ceramic sheet through the lead, the piezoelectric ceramic sheet vibrates, which further drives the vibration sheet to vibrate together. There are several micropores of uniform size distributed on the vibration sheet. Under the excitation of vibration, the liquid surface is continuously squeezed. After reaching a certain frequency and amplitude, part of the liquid in contact with the vibration sheet is squeezed out through the micropores to form tiny droplets, thus atomization is produced.

In the case of atomization, only 1-5 µm droplets among atomized droplets can enter the lungs and reach the lesion to achieve a therapeutic effect. The greater the proportion of 1-5µm droplets atomized, the better the treatment effect. At present, commercially available atomization apparatus include air compression type, oxygen compression type, and ultrasonic type. However, these atomization devices have the following disadvantages: the atomization rate (the amount of mist sprayed out per unit time) is as slow as about 2-3 mg/s, resulting in a long administration time and continuous atomization for 3-5 minutes; the operation of inhalation is cumbersome and the compliance is poor; during the administration process, the device is spraying regardless of whether the user inhales the mist droplets, causing the user to inhale the drug too much, and the drug is wasted seriously; due to the limitation of the atomization principle, after these devices increase the atomization speed of a drug, the proportion of 1-5µm droplets among atomized droplets will be greatly reduced, the proportion of drugs entering the lungs will be small, and the waste of drugs will increase. Therefore, a comprehensive design of a low-speed and long-life device is adopted.

The mesh-type atomization apparatus is an improved atomization apparatus based on the principle of ultrasonic atomization, but the design concept of commercially available products still follows the ideas of previous devices, namely, low atomization speed and long service life. So its atomization speed is also 2-3mg/s, and its service life is 3000-5000 hours. The important component of the existing mesh-type atomization apparatus, namely the atomization sheet, has an operating voltage that is much lower than its rated voltage to ensure its long life. Because the person skilled in the art believes that if the atomization sheet is operated under overload for a long time, the atomization sheet will be inevitably damaged. Therefore, the existing atomization apparatus does not work overload, and at the same time, it has the disadvantages of low atomization speed, long spray time, and poor treatment effect. For example, the atomization sheet with a rated voltage (Vpp) of 90 volts has an actual operating voltage of only 50-70 volts, and the more the operating voltage is lower than the rated voltage, the better. This design method of reserving safety windows is indeed a common sense in many product designs. In addition, the existing mesh-type atomization apparatus work continuously for a long time. If the atomization sheet is operated continuously for a long time with a high voltage greater than 70 volts, the ceramic on the atomization sheet will heat up, it is difficult to ensure the adhesive performance of the atomized sheet glue, and it is difficult to ensure the performance stability of the ceramic on the atomization sheet.

Based on the above, we need to design an atomization device and method that can overcome the above-mentioned technical prejudice and solve the above-mentioned problems.

### Summary of the invention

The object of the present invention is to provide an atomization apparatus, which can effectively increase the atomization speed of a drug.

Another object of the present invention is to provide a quick atomization method, which can effectively improve the atomization speed of a drug.

To achieve this, the present invention uses the following technical solutions:
in one aspect, an atomization apparatus is provided, which comprises:
an atomization sheet for atomizing a drug and having a rated voltage;
a voltage control element, electrically connected to the atomization sheet, for providing an operating voltage for the atomization sheet, wherein the operating voltage is not less than 70 V, and the operating voltage is 60% to 150% of the rated voltage of the atomization sheet.

Specifically, when the operating voltage of the atomization sheet is not less than 70V and is 60% to 150% of the rated voltage of the atomization sheet, the piezoelectric ceramic sheet of the atomization sheet receives a larger excitation electric signal, and the vibration of atomization sheet increase, which can significantly increase the atomization speed of a drug. Combining the spray with the patient's inhalation can shorten the administration time from minutes to seconds, which is extremely convenient to use. High voltage operation for a few seconds a day does not cause the atomization sheet to heat up, nor does it shorten the lifespan too much. When the operating voltage exceeds the rated voltage too much, droplets will be sprayed out from the atomization sheet, the atomization sheet become hot, the proportion of 1-5 µm droplets among atomized droplets decreases, and the atomization effect is poor. When the operating voltage does not exceed 150% of the rated voltage, the above situation can be avoided and a high atomization rate can be guaranteed. At present, since the atomizers used in the market need to reserve a safety window to ensure their long life, the operating voltage is less than 70V, and the atomization rate is low.

In another aspect, an atomization apparatus is provided, which comprises:
an atomization sheet for atomizing a drug and having a rated voltage;
a voltage control element, electrically connected to the atomization sheet, for providing an operating voltage for the atomization sheet, wherein the ratio of the operating voltage to the rated voltage of the atomization sheet is greater than 100% and not greater than 150%.

Specifically, when the ratio of the operating voltage to the rated voltage of the atomization sheet is greater than 100% and not greater than 150%, the atomization sheet can work under overload, thereby increasing the atomization rate. The rated voltage of the atomization sheet can be obtained from the product manual of the atomization sheet. When the operating voltage exceeds the rated voltage too much, droplets will be sprayed out from the atomization sheet, the atomization sheet become hot, the proportion of 1-5 µm droplets among atomized droplets decreases, and the atomization effect is poor. When the operating voltage does not exceed 150% of the rated voltage, the above situation can be avoided and a high atomization rate can be guaranteed. At present, those skilled in the art generally believe that if the voltage provided to the atomization sheet is higher than its rated voltage for a long time, the atomization performance of the atomization sheet will reduce or even damaged before the spray is completed. Therefore, the operating voltage of the atomizers used in the market is lower than the rated voltage, the atomization rate is low, and the spray time is long.

As a preferred technical solution of the atomization device, the voltage control element includes a power supply and a voltage control circuit. The input end of the voltage control circuit is electrically connected to the power supply. The output end of the voltage control circuit is electrically connected to the atomization sheet, and the voltage control circuit can perform step-up processing on the voltage provided by the power supply.

Specifically, by performing step-up processing on the voltage and inputting it to the atomization sheet, the output voltage of the power supply can be reduced, thereby making the use of the power supply more secure.

As a preferred technical solution of the atomization device, the voltage control circuit includes a voltage regulating element, and the voltage regulating element is used to change the operating voltage provided to the atomization sheet.

Specifically, the voltage control circuit is capable of performing step-up processing on the voltage provided by the power supply. In the prior art, the capability of step-up processing on the voltage of the voltage control circuit is fixed, that is, the step-up ratio is fixed. The voltage control circuit can only increase the voltage of the power supply to a fixed design value, which is the operating voltage, and can't choose and change the fixed design value according to factors such as different populations, different drugs, different symptoms, that is, the operating voltage cannot be changed, which limits the versatility of the atomization device. The voltage regulating element of this solution can control the voltage control circuit, so that the voltage subjected to the step-up processing of the voltage control circuit is larger than a fixed design value, and realize the adjustment of the operating voltage, thus the atomization device can meet the need of different populations, different drugs, and different symptoms, and then provide the versatility of the atomization device.

Further, the operating voltage can be selected and determined according to different populations, drugs, and symptoms, and the operating voltage can be selected and set by patients according to their needs during use. The operating voltage of this solution is not less than 70V, and the operating voltage is 60% ∼ 150% of the rated voltage of the atomization sheet, or the ratio of the operating voltage of this solution to the rated voltage of the atomization sheet is greater than 100% and not greater than 150%. By setting the voltage regulating element, the versatility of the product can be improved. In one aspect, it can reduce the product specifications of the enterprise and the product switching frequency during production, thus improve production efficiency and reduce product maintenance costs; in another aspect, it can meet the needs of different patients and improve convenience of use.

As a preferred technical solution of an atomization device, the voltage regulating element is a transformer, and the turns ratio of the primary coil and secondary coil of the transformer is adjustable.

Specifically, the turns ratio of the primary coil and secondary coil of the transformer is adjustable, that is, the step-up ratio of the voltage control circuit can be adjusted. When the output voltage of the power supply is fixed, the operating voltage can be adjusted to not less than 70V, and the operating voltage is 60% to 150% of the rated voltage of the atomization sheet, or the operating voltage can be adjusted to a ratio of the rated voltage of the atomization sheet greater than 100% and not greater than 150%, and the adjustment is convenient.

Preferably, the voltage control element further includes a voltage adjustment knob for manually adjusting the turns ratio of the primary coil and secondary coil of the transformer.

As a preferred technical solution of the atomization device, the atomization device further includes a time control element, which is electrically connected to the atomization sheet through a voltage control element, and is used to control the operating time of the atomization sheet.

Specifically, the atomization device of the present invention can perform multiple sprays. Before the devices leave the factory, the cumulative working time of the atomization sheet, the working time of the single spray of the atomization sheet, and the number of sprays are fixedly set, that is, the predetermined time limit and the number of sprays are set. The multiplier of the working time of the single spray of the atomization sheet and the number of sprays is equal to the cumulative working time of the atomization sheet. The time control element can control the cumulative working time of the atomization sheet and the working time of a single spray of the atomization sheet.

Preferably, the atomization device includes a casing and an atomization component. The atomization component is installed inside the casing, and the atomization sheet is installed inside the atomization component. The atomization component of the present invention is a structure for installing and fixing an atomization sheet, and includes a pipeline section having an inlet end and an outlet end, and the atomization sheet is installed in the pipeline section. An inlet end of the pipeline section is configured to be connected to a liquid storage part of the atomization device, and an outlet end of the pipeline section is in communication with a nozzle of the atomization device.

Further, the voltage control element is installed inside the casing and outside the atomization component, and the time control element is installed inside the casing and outside the atomization component.

Preferably, an on-off switch is provided between the atomization sheet and the voltage control element. The time control element is connected to the on-off switch. The time control element controls the closing or opening of the on-off switch to realize the closing or opening of the power transmission path between the atomization sheet and the voltage control element, and thus control the working time of the atomization sheet.

Preferably, an on-off switch is provided inside the voltage control element. The time control element is connected to the on-off switch. The time control element controls the closing or opening of the on-off switch to realize the operation or stop of the voltage control element, and thus control the working time of the atomization sheet.

As a preferred technical solution of the atomization device, the time control element adopts a crystal oscillator or a ceramic oscillator, and the crystal oscillator or the ceramic oscillator is electrically connected to the voltage control element. In addition to the two above, other components can be used for the time control element.

Preferably, the time control element is integrated on the voltage control circuit.

As a preferred technical solution of the atomization device, the operating time of each spray of the atomization sheet is 0.5 to 2 seconds.

Specifically, the operating time of each spray of the atomization sheet is within the patient's single breathing time, which is short, and the use is convenient.

As a preferred technical solution of the atomization device, the number of sprays of the atomization device is 30 to 240 times.

Specifically, the multiplier of the working time of the single spray of the atomization sheet and the number of sprays is equal to the cumulative working time of the atomization sheet. The cumulative working time of the atomization sheet is 15 to 480 seconds, which is short. The atomization sheet will not be damaged until the spray is completed.

Preferably, the working time of the single spray of the atomization sheet is 1.5 seconds, the number of sprays is 120, and the cumulative working time of the atomization sheet is 180 seconds.

In other aspect, a quick atomization method is provided in the present invention. A voltage control element provides an operating voltage for the atomization sheet, wherein the operating voltage is not less than 70 V, and the operating voltage is 60% to 150% of the rated voltage of the atomization sheet. The atomization sheet atomizes the drug under the operating voltage.

Specifically, when the operating voltage is not less than 70 V, and the operating voltage is 60% to 150% of the rated voltage of the atomization sheet, the atomization speed can be improved. Combining the spray with the patient's inhalation can shorten the administration time from minutes to seconds, which is extremely convenient to use. High voltage operation for a few seconds a day does not cause the atomization sheet to heat up, nor does it shorten the lifespan too much. When the operating voltage exceeds the rated voltage too much, droplets will be sprayed out from the atomization sheet, the atomization sheet become hot, the proportion of 1-5 µm droplets among atomized droplets decreases, and the atomization effect is poor. When the operating voltage does not exceed 150% of the rated voltage, the above situation can be avoided and a high atomization rate can be guaranteed. At present, since the atomizers used in the market need to reserve a safety window to ensure their long life, the operating voltage is less than 70V, and the atomization rate is low.

In other aspect, a quick atomization method is provided in the present invention. A voltage control element provides an operating voltage for the atomization sheet, wherein the ratio of the operating voltage to the rated voltage of the atomization sheet is greater than 100% and not greater than 150%. The atomization sheet atomizes the drug under the operating voltage.

Specifically, the ratio of the operating voltage to the rated voltage of the atomization sheet is greater than 100% and not greater than 150%, which enables the atomization sheet to work overload, thereby increasing the atomization speed. The rated voltage of the atomization sheet can be obtained from the product manual of the atomization sheet. When the operating voltage exceeds the rated voltage too much, droplets will be sprayed out from the atomization sheet, the atomization sheet become hot, the proportion of 1-5 µm droplets among atomized droplets decreases, and the atomization effect is poor. When the operating voltage does not exceed 150% of the rated voltage, the above situation can be avoided and a high atomization rate can be guaranteed. At present, those skilled in the art generally believe that if the voltage provided to the atomization sheet is higher than its rated voltage for a long time, the atomization performance of the atomization sheet will reduce or even damaged before the spray is completed. Therefore, the operating voltage of the atomizers used in the market is lower than the rated voltage, the atomization rate is low, and the spray time is long.

As a preferred technical solution of a quick atomization method, the quick atomization method specifically includes the following steps:
the power supply provides voltage to the voltage control circuit;
the voltage control circuit performs step-up processing on the voltage provided by the power supply, which is then transformed into an operating voltage. The operating voltage is not less than 70V, and the operating voltage is 60% ∼ 150% of the rated voltage of the atomization sheet, or the ratio of the operating voltage to the rated voltage of the atomization sheet is greater than 100% and not greater than 150%.

The voltage control circuit sends the operating voltage to the atomization sheet, and the atomization sheet atomizes the drug under the operating voltage.

As a preferred technical solution of a quick atomization method, the voltage control circuit performs step-up processing on the voltage provided by the power supply, which includes the following steps:
regulating the voltage regulating element in the voltage control circuit to change the operating voltage provided to the atomization sheet.

Specifically, the output voltage of the power supply is fixed, by regulating the voltage regulating element, the voltage control circuit is controlled so that the operating voltage can be adjusted to obtain different atomization speeds, thus the atomization device can meet the need of different populations, different drugs, and different symptoms, and then provide the versatility of the atomization device.

As a preferred technical solution of a quick atomization method, when adjusting a voltage regulating element in a voltage control circuit, the method includes the following steps:
the turns ratio of the primary and secondary coil of the voltage regulating element is regulated to change the operating voltage provided to the atomization sheet, wherein the voltage adjustment element is a transformer.

Specifically, the voltage control element further includes a voltage adjustment knob for manually adjusting the turns ratio of the primary and secondary coil of the transformer, that is, adjusting the step-up ratio of the voltage control circuit. So that in the condition that the output voltage of the power supply is fixed, the operating voltage can be adjusted to not less than 70V, and the operating voltage is 60% to 150% of the rated voltage of the atomization sheet, or the operating voltage can be adjusted to a ratio of the rated voltage of the atomization sheet greater than 100% and not greater than 150%.

As a preferred technical solution of a rapid atomization method, before the power supply provides voltage to the voltage control circuit, the method further includes the following steps:
determining the step-up ratio of the voltage control circuit and determining the rated voltage of the atomization sheet;
selecting a power supply with a suitable output voltage according to the step-up ratio and rated voltage to ensure that after the output voltage of the power supply is subjected to the step-up processing of the voltage control circuit, the operating voltage provided to the atomization sheet is not less than 70V, and the operating voltage is 60% to 150% of the rated voltage of the atomization sheet, or the operating voltage can be adjusted to a ratio of the rated voltage of the atomization sheet greater than 100% and not greater than 150%.

Specifically, the step-up ratio of the voltage-control circuit is fixed. The step-up ratio of the voltage-control circuit can be obtained from the product manual of the voltage-control circuit board. The rated voltage of the atomization can be obtained from the product manual of the atomization sheet.

As a preferred technical solution of a quick atomization method, while executing a voltage control circuit to input an operating voltage to the atomization sheet, the method further includes the following steps:
starting the timer;
determining whether the cumulative working time of the atomization sheet has reached the predetermined time limit, if so, stopping inputting the operating voltage to the atomization sheet to stop the atomization sheet from performing atomization treatment on the drug; if not, whether the working time of a single spray of the atomization sheet has reached the predetermined time limit need to be determined, if yes, stopping inputting the operating voltage to the atomization sheet to stop the atomization sheet from performing atomization treatment on the drug; if not, returning to determine whether the working time of a single spray of the atomization sheet has reached the predetermined time limit.

Specifically, the atomization device of the present invention can perform multiple sprays. Before the devices leave the factory, the cumulative working time of the atomization sheet and the working time of the single spray of the atomization sheet are fixedly set, that is, the predetermined time limit is set. The time control element can control the cumulative working time of the atomization sheet and the working time of a single spray of the atomization sheet.

Specifically, timing and judging whether the working time reaches a predetermined time limit are performed by a time control element.

As a preferred technical solution of a quick atomization method, determining whether the working time of the atomization sheet has reached a predetermined time limit, specifically:
counting time in reverse order, setting the initial time of the countdown clock to the value of the predetermined time limit, and determining whether the working time of the atomization sheet is equal to zero;
alternatively, counting time in positive order, setting the initial time of the positive sequence timing to be zero, and determining whether the working time of the atomization sheet is greater than or equal to a value of a predetermined time limit.

Specifically, determining the working time of the atomization sheet includes determining the cumulative working time of the atomization sheet and the working time of a single spray of atomization sheet.

As a preferred technical solution of a quick atomization method, the operating time of each spray of the atomization sheet is 0.5 to 2 seconds, the number of sprays of the atomization device is 30 to 240 times.

Preferably, the number of sprays of the atomization device is 120, the cumulative working time of the atomization sheet is 180 seconds, and the working time of the single spray of the atomization sheet is set to be 1.5 seconds.

Specifically, the multiplier of the working time of the single spray of the atomization sheet and the number of sprays of the atomization device is equal to the cumulative working time of the atomization sheet. The cumulative working time of the atomization sheet is 15 to 480 seconds, which is short. The atomization sheet will not be damaged before the spray is completed.

Preferably, the working time of the single spray of the atomization sheet is 1.5 seconds, the number of sprays of the atomization device is 120, and the cumulative working time of the atomization sheet is 180 seconds.

The beneficial effects of the present invention are:
an atomization apparatus is provided. An operating voltage provided for the atomization sheet is not less than 70 V, and the operating voltage is 60% to 150% of the rated voltage of the atomization sheet, or the ratio of the operating voltage to the rated voltage of the atomization sheet is greater than 100% and not greater than 150%. So that the atomization speed of a drug can be significantly improved, and the proportion of 1 to 5 µm droplets among atomized droplets remains unchanged. A quick atomization method is provided. An operating voltage provided for the atomization sheet is not less than 70 V, and the operating voltage is 60% to 150% of the rated voltage of the atomization sheet, or the ratio of the operating voltage to the rated voltage of the atomization sheet is greater than 100% and not greater than 150%. So that the atomization speed of a drug can be significantly improved, and the proportion of 1 to 5 µm droplets among atomized droplets remains unchanged.

At the same time, the atomization device and the quick atomization method of the present invention perform multiple sprays, each spraying time is short, lasting only a few seconds, even if the atomization sheet works overload, the atomization sheet will not be damaged before reaching the preset limited number of uses. The atomization device and the quick atomization method have the advantages of high atomization speed, short spray time, and good treatment effect.

### Description of the drawings

In order to more clearly illustrate the specific embodiments of the present invention or the technical solutions in the prior art, the drawings needed to be used in the specific embodiments or the description of the prior art are briefly introduced below. Obviously, the drawings in the following description are some embodiments of the present invention. For those skilled in the art, other drawings can be obtained according to these drawings without creative efforts.
Figure 1 is the flow chart of quick atomization method of Example 1.
Figure 2 is the flow chart of quick atomization method of Example 2.
Figure 3 is the flow chart of quick atomization method of Example 7.
Figure 4 is the composition schematic diagram of atomization apparatus of Example 7.

### Examples

The technical solution of the present invention will be clearly and completely described below with reference to the accompanying drawings. Obviously, the described embodiments are part embodiments of the present invention, but not all embodiments. Based on the embodiments of the present invention, all other embodiments obtained by the skilled in the art without creative efforts shall fall within the protection scope of the present invention.

In the description of the present invention, it should be noted that the orientations or positional relationships indicated by the terms "center", "up", "down", "left", "right", "vertical", "horizontal", "inside", "outside", *etc.,* are based on the orientations or positional relationships shown in the drawings, and are merely for the convenience of describing the present invention and simplify the description, rather than indicating or implying that the device or element referred to must have a particular orientation, be constructed and operate in a particular orientation, and therefore should not be construed as limiting the invention. In addition, the terms "first", "second", and "third" are used for descriptive purposes only, and should not be construed to indicate or imply relative importance.

In the description of the present invention, it should be noted that the terms "installation", "connected", and "connection" should be understood in a broad sense unless otherwise specified and limited. For example, it can be a fixed connection, a detachable connection, or an integral connection; it can be a mechanical connection or an electrical connection; it can be directly connected, or it can be indirectly connected through an intermediate medium, or it can be the internal connection of two elements. For those skilled in the art, the specific meanings of the above terms in the present invention can be understood on a case-by-case basis.

In order to make the structural features and achieved effects of the present invention be further understood and recognized, the preferred embodiments and drawings are used in conjunction with the detailed description, as follows:

### Example 1

An atomization apparatus comprises:
an atomization sheet for atomizing a drug and having a rated voltage;
a voltage control element, electrically connected to the atomization sheet, for providing an operating voltage for the atomization sheet, wherein the operating voltage is not less than 70 V, and the operating voltage is 60% to 150% of the rated voltage of the atomization sheet.

When the operating voltage is not less than 70 V, and the operating voltage is 60% to 150% of the rated voltage of the atomization sheet, the atomization speed can be improved. Combining the spray with the patient's inhalation can shorten the administration time from minutes to seconds, which is extremely convenient to use. High voltage operation for a few seconds a day does not cause the atomization sheet to heat up, nor does it shorten the lifespan too much. When the operating voltage exceeds the rated voltage too much, droplets will be sprayed out from the atomization sheet, the atomized sheet become hot, the proportion of 1-5 µm droplets among atomized droplets decreases, and the atomization effect is poor. When the operating voltage does not exceed 150% of the rated voltage, the above situation can be avoided and a high atomization rate can be guaranteed.

In this example, the voltage control element includes a power supply and a voltage control circuit. One end of the voltage control circuit is electrically connected to the power supply, and the other end of the voltage control circuit is electrically connected to the atomization sheet. The voltage control circuit performs step-up processing on the voltage provided by the power supply. By performing step-up processing on the voltage and inputting it to the atomization sheet, the output voltage of the power supply can be reduced, thereby making the use of the power supply more secure.

In this example, the voltage control circuit includes a voltage regulating element, and the voltage regulating element is used to change the operating voltage provided to the atomization sheet. The voltage regulating element is provided with a regulating device on the casing.

In this example, the voltage regulating element is a transformer, and the turns ratio of the primary coil and secondary coil of the transformer is adjustable. That is, the step-up ratio of the voltage control circuit is adjustable. When the output voltage of the power supply is fixed, the operating voltage can be adjusted to not less than 70V, and the operating voltage is 60% ∼ 150% of the rated voltage of the atomization sheet, which is easy to adjust.

In this example, the voltage control circuit further includes voltage adjustment knob for manually adjusting the turns ratio of the primary and secondary coil of the transformer. The voltage adjustment knob is set on the casing and can be manually adjusted by turning.

The atomization device includes a casing and an atomization component. The atomization component is installed inside the casing, and the atomization sheet is installed inside the atomization component. Both the power supply and the voltage control circuit are installed inside the casing and located outside the atomization component.

A quick atomization method based on the atomization apparatus above is provided in this example. The voltage control element provides an operating voltage to the atomization sheet, wherein the operating voltage is not less than 70 V, and the operating voltage is 60% to 150% of the rated voltage of the atomization sheet. The atomization sheet atomizes the drug under the operating voltage.

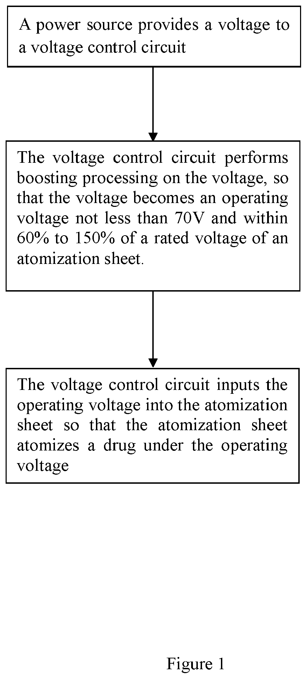

In this example, as shown in Figure 1, the quick atomization method includes the following steps:
the power supply provides voltage to the voltage control circuit;
the voltage control circuit performs step-up processing on the voltage provided by the power supply, which is then transformed into an operating voltage. The operating voltage is not less than 70V, and the operating voltage is 60% ∼ 150% of the rated voltage of the atomization sheet;
the voltage control circuit sends the operating voltage to the atomization sheet, and the atomization sheet atomizes the drug under the operating voltage.

In this example, the voltage control circuit performs step-up processing on the voltage provided by the power supply, which includes the following steps:
regulating the voltage regulating element in the voltage control circuit to change the operating voltage provided to the atomization sheet.

In this example, when adjusting a voltage regulating element in a voltage control circuit, the method includes the following steps:
the turns ratio of the primary and secondary coil of the voltage regulating element is regulated to change the operating voltage provided to the atomization sheet, wherein the voltage regulating element is a transformer.

In this example, the power supply is selected first, and then a voltage control circuit with a suitable step-up ratio is selected. The output voltage of the power supply is fixed, by regulating the voltage regulating element, the voltage control circuit is controlled so that the operating voltage can be adjusted to not less than 70, and the operating voltage is 60% to 150% of the rated voltage of the atomization sheet, thus the atomization device can meet the need of different populations, different drugs, and different symptoms, and then provide the versatility of the atomization device.

The voltage control element further includes a voltage adjustment knob for manually adjusting the turns ratio of the primary and secondary coil of the transformer, that is, adjusting the step-up ratio of the voltage control circuit. So that in the condition that the output voltage of the power supply is fixed, the operating voltage can be adjusted to not less than 70V, and the operating voltage is 60% to 150% of the rated voltage of the atomization sheet.

### Example 2

An atomization apparatus comprises:
an atomization sheet for atomizing a drug and having a rated voltage;
a voltage control element, electrically connected to the atomization sheet, for providing an operating voltage for the atomization sheet, wherein the ratio of the operating voltage to the rated voltage of the atomization sheet is greater than 100% and not greater than 150%.

When the ratio of the operating voltage to the rated voltage of the atomization sheet is greater than 100% and not greater than 150%, the atomization sheet can work under overload, thereby increasing the atomization rate. The rated voltage of the atomization sheet can be obtained from the product manual of the atomization sheet. When the operating voltage exceeds the rated voltage too much, droplets will be sprayed out from the atomized sheet, the atomized sheet become hot, the proportion of 1-5 µm droplets among atomized droplets decreases, and the atomization effect is poor. When the operating voltage does not exceed 150% of the rated voltage, the above situation can be avoided and a high atomization rate can be guaranteed.

In this example, the voltage control element includes a power supply and a voltage control circuit. The input end of the voltage control circuit is electrically connected to the power supply. The output end of the voltage control circuit is electrically connected to the atomization sheet, and the voltage control circuit can perform step-up processing on the voltage provided by the power supply. By performing step-up processing on the voltage and inputting it to the atomization sheet, the output voltage of the power supply can be reduced, thereby making the use of the power supply more secure.

In this example, the voltage control circuit includes a voltage regulating element, and the voltage regulating element is used to change the operating voltage provided to the atomization sheet. The voltage regulating element is provided with a regulating device on the casing.

In this example, the voltage regulating element is a transformer, and the turns ratio of the primary coil and secondary coil of the transformer is adjustable. That is, the step-up ratio of the voltage control circuit can be adjusted. When the output voltage of the power supply is fixed, the operating voltage can be adjusted to a ratio of the rated voltage of the atomization sheet greater than 100% and not greater than 150%.

In this example, the voltage control element further includes a voltage adjustment knob for manually adjusting the turns ratio of the primary coil and secondary coil of the transformer. The voltage adjustment knob is set on the casing and can be manually adjusted by turning.

The atomization device includes a casing and an atomization component. The atomization component is installed inside the casing, and the atomization sheet is installed inside the atomization component. Both the power supply and the voltage control circuit are installed inside the casing and located outside the atomization component.

A quick atomization method based on the atomization apparatus above is provided in this example. The voltage control element provides an operating voltage to the atomization sheet, wherein the ratio of the operating voltage to the rated voltage of the atomization sheet is greater than 100% and not greater than 150%. The atomization sheet atomizes the drug under the operating voltage.

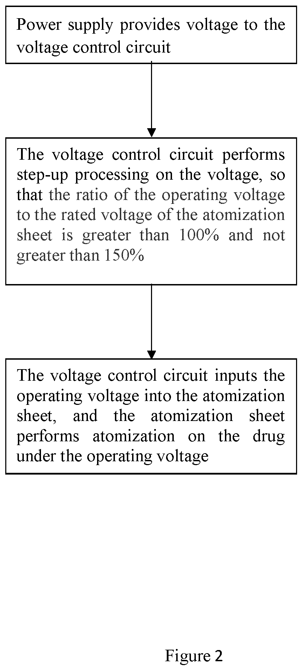

In this example, as shown in Figure 2, the quick atomization method includes the following steps:
the power supply provides voltage to the voltage control circuit;
the voltage control circuit performs step-up processing on the voltage provided by the power supply, which is then transformed into an operating voltage. The ratio of the operating voltage to the rated voltage of the atomization sheet is greater than 100% and not greater than 150%;
the voltage control circuit sends the operating voltage to the atomization sheet, and the atomization sheet atomizes the drug under the operating voltage.

In this example, the voltage control circuit performs step-up processing on the voltage provided by the power supply, which includes the following steps:
regulating the voltage regulating element in the voltage control circuit to change the operating voltage provided to the atomization sheet.

In this example, when adjusting a voltage regulating element in a voltage control circuit, the method includes the following steps:
the turns ratio of the primary and secondary coil of the voltage regulating element is regulated to change the operating voltage provided to the atomization sheet, wherein the voltage adjustment element is a transformer.

In this example, the power supply is selected first, and then a voltage control circuit with a suitable step-up ratio is selected. The output voltage of the power supply is fixed, by regulating the voltage regulating element, the voltage control circuit is controlled so that the ratio of the operating voltage to the rated voltage of the atomization sheet can be adjusted to greater than 100% and not greater than 150%, thus the atomization device can meet the need of different populations, different drugs, and different symptoms, and then provide the versatility of the atomization device.

The voltage control element further includes a voltage adjustment knob for manually adjusting the turns ratio of the primary and secondary coil of the transformer, that is, adjusting the step-up ratio of the voltage control circuit. So that in the condition that the output voltage of the power supply is fixed, the ratio of the operating voltage to the rated voltage of the atomization sheet can be adjusted to greater than 100% and not greater than 150%.

### Example 3

By using the atomization device and the quick atomization method in Example 1 or Example 2, the atomization sheets with a rated voltage of 90V commonly used in the market are tested. The results are shown in Table 1 below.

**Table 1**

| The rated voltage of the atomizati on sheet(v) | Operating voltage(V ) | The ratio of the operating voltage to the rated voltage(% ) | Atomizatio n speed(mg/s ) | Moveme nt speed of the mist (cm/s) | The proportio n of 1 to 5 um droplets | The cumulativ e working time(s) | When the cumulativ e working time is reached, whether working is normal? |
|---|---|---|---|---|---|---|---|
| 90 | 45.04 | 50 | 2.05 | 4.58 | 46.42 | 900 | Normal |
| | 54.12 | 60 | 3.44 | 11.32 | 46.35 | 900 | Normal |
| | 63.13 | 70 | 4.98 | 12.58 | 46.44 | 900 | Normal |
| | 70.00 | 78 | 8.05 | 12.83 | 45.95 | 900 | Normal |
| | 72.02 | 80 | 8.12 | 13.94 | 45.91 | 900 | Normal |
| | 81.07 | 90 | 9.47 | 14.01 | 45.87 | 900 | Normal |
| | 90.10 | 100 | 11.37 | 16.53 | 45.81 | 900 | Normal |
| | 99.04 | 110 | 13.62 | 18.84 | 45.22 | 900 | Normal |
| | 108.07 | 120 | 14.59 | 19.74 | 45.16 | 900 | Normal |
| | 116.9 | 130 | 17.13 | 21.95 | 45.18 | 900 | Normal |
| | 126.02 | 140 | 19.85 | 23.78 | 45.38 | 900 | Normal |
| | 135.00 | 150 | 22.46 | 25.13 | 45.11 | 900 | Normal |

All of the atomization sheets used in the table above are the atomization sheets with a rated voltage of 90V commonly used in the market. From the test results in the table above, it can be seen that when the working voltage provided to the atomization sheet is not less than 70V, and the working voltage is 60% ∼ 150% of the rated voltage of the atomization sheet, or when the ratio of the operating voltage to the rated voltage of the atomization sheet is greater than 100% and not greater than 150%, the atomization speed of the drug and the movement speed of the mist are effectively improved, especially the atomization speed is significantly improved, and the proportion of 1 to 5 µm droplets among atomized droplets remains almost unchanged. When the ratio of the operating voltage to the rated voltage of the atomization sheet is not greater than 150%, the atomization sheet will not be damaged when the cumulative working time is reached, namely when the total sprays are finished. The atomization sheet can be sprayed normally, there are no large droplets, and the atomization sheet is not hot.

### Example 4

The difference between Example 3 and the Example 4 is that:
the atomization sheets with a rated voltage of 120V commonly used in the market are tested. The results are shown in Table 2 below.

**Table 2**

| The rated voltage of the atomization sheet(v) | Operating voltage(V ) | The ratio of the operating voltage to the rated voltage(% ) | Atomization speed(mg/s ) | Movement speed of the mist (cm/s) | The proportion of 1 to 5 um droplets | The cumulative working time(s) | When the cumulative working time is reached, whether working is normal? |
|---|---|---|---|---|---|---|---|
| 120 | 60.04 | 50 | 4.51 | 11.52 | 46.32 | 900 | Normal |
| | 70.00 | 58 | 8.08 | 12.91 | 46.28 | 900 | Normal |
| | 72.08 | 60 | 8.43 | 13.48 | 46.87 | 900 | Normal |
| | 84.11 | 70 | 9.71 | 15.47 | 46.21 | 900 | Normal |
| | 95.95 | 80 | 11.96 | 16.04 | 45.98 | 900 | Normal |
| | 108.07 | 90 | 14.55 | 18.12 | 45.62 | 900 | Normal |
| | 120.01 | 100 | 18.17 | 19.87 | 45.81 | 900 | Normal |
| | 132.08 | 110 | 21.00 | 23.11 | 45.69 | 900 | Normal |
| | 144.17 | 120 | 23.48 | 25.52 | 45.78 | 900 | Normal |
| | 156.09 | 130 | 25.79 | 27.25 | 45.85 | 900 | Normal |
| | 167.89 | 140 | 28.42 | 29.45 | 45.33 | 900 | Normal |
| | 180.07 | 150 | 31.87 | 32.87 | 45.28 | 900 | Normal |

All of the atomization sheets used in the table above are the atomization sheets with a rated voltage of 120V commonly used in the market. From the test results in the table above, it can be seen that when the working voltage provided to the atomization sheet is not less than 70V, and the working voltage is 60% ∼ 150% of the rated voltage of the atomization sheet, or when the ratio of the operating voltage to the rated voltage of the atomization sheet is greater than 100% and not greater than 150%, the atomization speed of the drug and the movement of the mist are effectively improved, especially the atomization speed is significantly improved, and the proportion of 1 to 5 µm droplets among atomized droplets remains almost unchanged. When the ratio of the operating voltage to the rated voltage of the atomization sheet is not greater than 150%, the atomization sheet will not be damaged when the cumulative working time is reached, namely when the total sprays are finished. The atomization sheet can be sprayed normally, there are no large droplets, and the atomization sheet is not hot.

### Example 5

The difference between this example and the Example 1 is that:
in this example, before the power supply provides voltage to the voltage control circuit, the following steps are further included:
determining the step-up ratio of the voltage control circuit, and determining the rated voltage of the atomization sheet;
selecting a power supply with a suitable output voltage according to the step-up ratio and rated voltage to ensure that after the output voltage of the power supply is subjected to the step-up processing of the voltage control circuit, the operating voltage provided to the atomization sheet is not less than 70V, and the operating voltage is 60% to 150% of the rated voltage of the atomization sheet.

In this example, the power supply is selected first, and then the power supply with the appropriate output voltage is selected. The turns ratio of the primary coil and the secondary coil of the transformer is not adjustable. The step-up ratio of the voltage control circuit is fixed. The step-up ratio of the voltage control circuit can be obtained from the product manual of the voltage control circuit board. The rated voltage of the atomization sheet can be obtained from the product manual of the atomization sheet.

### Example 6

The difference between this example and the Example 2 is that:
in this example, before the power supply provides voltage to the voltage control circuit, the following steps are further included:
determining the step-up ratio of the voltage control circuit, and determining the rated voltage of the atomization sheet;
selecting a power supply with a suitable output voltage according to the step-up ratio and rated voltage to ensure that after the output voltage of the power supply is subjected to the step-up processing of the voltage control circuit, the ratio of the operating voltage to the rated voltage of the atomization sheet is greater than 100% and not greater than 150%.

In this example, the power supply is selected first, and then the power supply with the appropriate output voltage is selected. The turns ratio of the primary coil and the secondary coil of the transformer is not adjustable. The step-up ratio of the voltage control circuit is fixed. The step-up ratio of the voltage control circuit can be obtained from the product manual of the voltage control circuit board. The rated voltage of the atomization sheet can be obtained from the product manual of the atomization sheet.

### Example 7

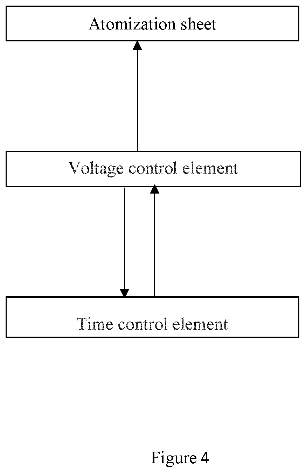

The difference between this example and Example 1 or Example 2 is that:
as shown in Figure 4, the atomization device further includes a time control element, which is electrically connected to the atomization sheet through a voltage control element, and is used to control the operating time of the atomization sheet.

The atomization device of the present invention can perform multiple sprays. Before the devices leave the factory, the cumulative working time of the atomization sheet, the working time of the single spray of the atomization sheet, and the number of sprays are fixedly set, that is, the predetermined time limit and the number of sprays are set. The multiplier of the working time of the single spray of the atomization sheet and the number of sprays is equal to the cumulative working time of the atomization sheet. The time control element can control the cumulative working time of the atomization sheet and the working time of a single spray of the atomization sheet.

In this example, the voltage control element is installed inside the casing and outside the atomization component, and the time control element is installed inside the casing and outside the atomization component.

In this example, an on-off switch is provided inside the voltage control element. The time control element is connected to the on-off switch. The time control element controls the closing or opening of the on-off switch to realize the operation or stop of the voltage control element, and thus control the working time of the atomization sheet.

In other example, an on-off switch is provided between the atomization sheet and the voltage control element. The time control element is connected to the on-off switch. The time control element controls the closing or opening of the on-off switch to realize the closing or opening of the power transmission path between the atomization sheet and the voltage control element, and thus control the working time of the atomization sheet.

In this example, the time control element includes a crystal oscillator or a ceramic oscillator, and the crystal oscillator or the ceramic oscillator is electrically connected to the voltage control element.

In this example, the time control element is integrated on the voltage control circuit.

In this example, the operating time of each spray of the atomization sheet is 1.5 seconds, the number of sprays of the atomization device is 120 times, and the cumulative working time of the atomization sheet is 15 to 480 seconds, wherein the multiplier of the working time of the single spray of the atomization sheet and the number of sprays is equal to the cumulative working time of the atomization sheet.

Since the cumulative working time of the atomization sheet is short, the atomization sheet will not be damaged when the spray is completed, that is, the spray can be completed before the atomization sheet is damaged, and the spraying time is short and the use is convenient.

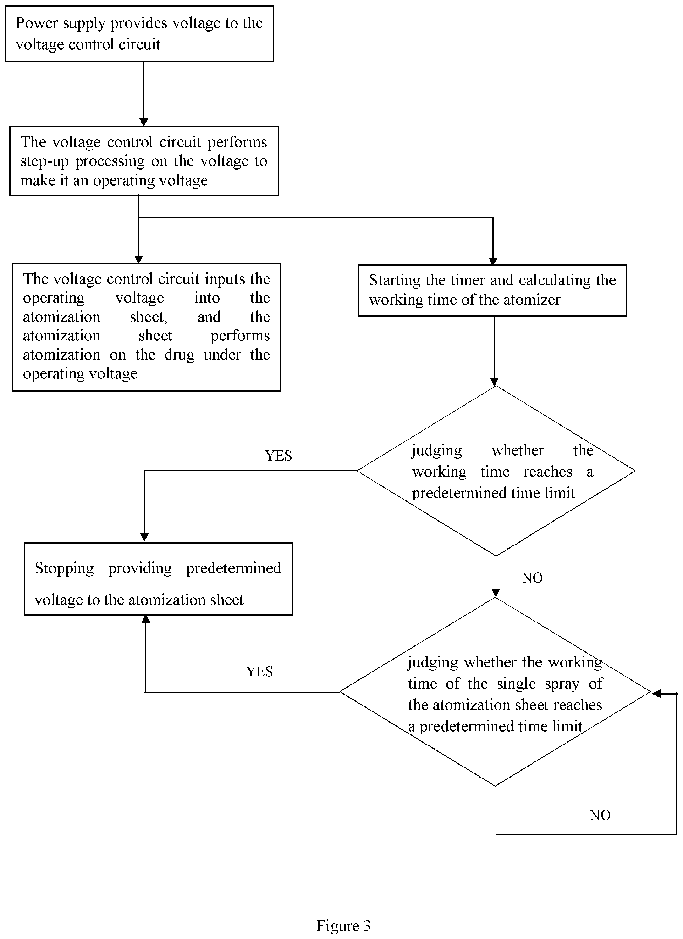

In this example, as shown in Figure 3, while executing a voltage control circuit to input an operating voltage to the atomization sheet, the following steps are further included:
starting the timer;
determining whether the cumulative working time of the atomization sheet has reached the predetermined time limit, if so, stopping inputting the operating voltage to the atomization sheet to stop the atomization sheet from performing atomization treatment on the drug; if not, whether the working time of a single spray of the atomization sheet has reached the predetermined time limit need to be determined, if yes, stopping inputting the operating voltage to the atomization sheet to stop the atomization sheet from performing atomization treatment on the drug; if not, returning to determine whether the working time of a single spray of the atomization sheet has reached the predetermined time limit.

In this example, determining whether the working time of the atomization sheet has reached a predetermined time limit, specifically:
counting time in reverse order, setting the initial time of the countdown clock to the value of the predetermined time limit, and determining whether the working time of the atomization sheet is equal to zero.

### Example 8

The difference between this example and the Example 7 is that:
in this example, determining whether the working time of the atomization sheet has reached a predetermined time limit, specifically:
counting time in positive order, setting the initial time of the positive sequence timing to be zero, and determining whether the working time of the atomization sheet is greater than or equal to the value of a predetermined time limit.

In this example, the operating time of each spray of the atomization sheet is 0.5 seconds, the number of sprays of the atomization device is 30 times, the cumulative working time of the atomization sheet is 15 seconds.

In other example, the operating time of each spray of the atomization sheet is 1 seconds, the number of sprays of the atomization device is 30 times, the cumulative working time of the atomization sheet is 30 seconds.

In other example, the operating time of each spray of the atomization sheet is 1 seconds, the number of sprays of the atomization device is 60 times, the cumulative working time of the atomization sheet is 60 seconds.

In other example, the operating time of each spray of the atomization sheet is 1.5 seconds, the number of sprays of the atomization device is 60 times, the cumulative working time of the atomization sheet is 90 seconds.

In other example, the operating time of each spray of the atomization sheet is 1.5 seconds, the number of sprays of the atomization device is 120 times, the cumulative working time of the atomization sheet is 180 seconds.

In other example, the operating time of each spray of the atomization sheet is 2 seconds, the number of sprays of the atomization device is 60 times, the cumulative working time of the atomization sheet is 120 seconds.

In other example, the operating time of each spray of the atomization sheet is 2 seconds, the number of sprays of the atomization device is 120 times, the cumulative working time of the atomization sheet is 240 seconds.

In other example, the operating time of each spray of the atomization sheet is 2 seconds, the number of sprays of the atomization device is 240 times, the cumulative working time of the atomization sheet is 480 seconds.

The parts that are not involved in the present invention are the same as the existing technology or can be implemented by using the existing technology, and are not repeated herein.

Finally, it should be noted that for those skilled in the art, it is obvious that the present application is not limited to the details of the above-mentioned exemplary embodiments, and can be implemented in other specific forms without departing from the spirit or basic features of the present application. Therefore, the embodiments should be regarded as exemplary and non-limiting in every respect. The scope of the present application is defined by the appended claims rather than the foregoing description, and therefore it is intended to include within the present application all changes that fall within the meaning and scope of equivalents of the claims.

In addition, it should be understood that although this specification is described in terms of embodiments, not every embodiment includes only an independent technical solution. This description of the specification is for clarity only, and those skilled in the art should take the specification as a whole. The technical solutions in the embodiments can also be appropriately combined to form other implementations that can be understood by those skilled in the art.

## Claims

1. An atomization apparatus, comprising:
an atomization sheet for atomizing a drug and having a rated voltage;
a voltage control element, electrically connected to the atomization sheet, for providing an operating voltage for the atomization sheet, wherein the operating voltage is not less than 70 V, and the operating voltage is 60% to 150% of the rated voltage of the atomization sheet.

2. An atomization apparatus, comprising:
an atomization sheet for atomizing a drug and having a rated voltage;
a voltage control element, electrically connected to the atomization sheet, for providing an operating voltage for the atomization sheet, wherein the ratio of the operating voltage to the rated voltage of the atomization sheet is greater than 100% and not greater than 150%.

3. The atomization apparatus of any one of claims 1 or 2, wherein the voltage control element comprises a power supply and a voltage control circuit, the input terminal of the voltage control circuit is electrically connected to the power supply, and the output terminal of the voltage control circuit is electrically connected to the atomization sheet, and the voltage control circuit can perform step-up processing on the voltage provided by the power supply.

4. The atomization apparatus of any one of claims 1 to 3, wherein the voltage control circuit comprises a voltage regulating element, for changing the operating voltage provided to the atomization sheet.

5. The atomization apparatus of any one of claims 1 to 4, wherein the voltage regulating element is a transformer, and the turns ratio of the primary coil and the secondary coil of the transformer is adjustable.

6. The atomization apparatus of any one of claims 1 to 5, wherein the voltage control element further comprises a voltage adjustment knob for manually adjusting the turns ratio of the primary and secondary coil of the transformer.

7. The atomization apparatus of any one of claims 1 to 6, wherein the atomization apparatus further comprises:
a time control element, electrically connected to the atomization sheet through the voltage control element, for controlling the operating time of the atomization sheet.

8. The atomization apparatus of any one of claims 1 to 7, wherein the operating time of each spray of the atomization sheet is 0.5 to 2 seconds.

9. A quick atomization method based on the atomization apparatus according to any one of claims 1, 3 to 8, wherein the operating voltage is not less than 70 V, the operating voltage is 60% to 150% of a rated voltage of the atomization sheet, and the atomization sheet performs atomizing treatment on the drug under the operating voltage.

10. A quick atomization method based on the atomization apparatus according to any one of claims 2, 3 to 8, wherein the voltage control element provides an operating voltage to the atomization sheet, the ratio of the operating voltage to the rated voltage of the atomization sheet is greater than 100% and not greater than 150%, and the atomization sheet performs atomizing treatment on the drug under the operating voltage.

11. A quick atomization method of any one of claims 9 or 10, wherein the number of sprays of the atomization apparatus is 30 to 240 times.
